(19) **Europäisches Patentamt European Patent Office Office européen des brevets**

(11) **EP 3 007 614 B1**

(12) **FASCICULE DE BREVET EUROPEEN**

(45) Date de publication et mention de la délivrance du brevet:
**08.11.2023 Bulletin 2023/45**

(21) Numéro de dépôt: **14737284.1**

(22) Date de dépôt: **12.06.2014**

(51) Classification Internationale des Brevets (IPC):
**A61B 5/00** (2006.01)    **A61B 5/024** (2006.01)
**A61B 5/0205** (2006.01)

(52) Classification Coopérative des Brevets (CPC):
**A61B 5/4824; A61B 5/02405; A61B 5/4821;
A61B 5/4839;** A61B 5/0205

(86) Numéro de dépôt international:
**PCT/FR2014/051436**

(87) Numéro de publication internationale:
**WO 2014/199093 (18.12.2014 Gazette 2014/51)**

(54) **DISPOSITIF D'ÉVALUATION DES BESOINS EN MÉDICAMENTS ET OU EN SOINS PARAMÉDICAUX, PROCÉDÉ D'ÉVALUATION POUR LA MISE EN OEUVRE DU DISPOSITIF D'ÉVALUATION ET DISPOSITIF DE DÉLIVRANCE ASSOCIÉ**

VORRICHTUNG ZUR BEURTEILUNG VON ARZNEI- UND/ODER PARAMEDIZINISCHEM PFLEGEBEDARF, BEURTEILUNGSVERFAHREN ZUR IMPLEMENTIERUNG DER BEURTEILUNGSVORRICHTUNG UND ZUGEHÖRIGE ABGABEVORRICHTUNG

DEVICE FOR ASSESSING MEDICATION AND/OR PARAMEDICAL CARE REQUIREMENTS, ASSESSMENT METHOD FOR IMPLEMENTING THE ASSESSMENT DEVICE, AND ASSOCIATED DELIVERY DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **14.06.2013 FR 1301377**

(43) Date de publication de la demande:
**20.04.2016 Bulletin 2016/16**

(73) Titulaire: **CENTRE HOSPITALIER REGIONAL UNIVERSITAIRE DE LILLE**
**59000 Lille (FR)**

(72) Inventeurs:
* **LOGIER, Regis**
  **59520 Marquette (FR)**
* **DELECROIX, Michel**
  **59139 Wattignies (FR)**
* **DE JONCKHEERE, Julien**
  **59160 Lomme (FR)**
* **JEANNE, Mathieu**
  **59800 Lille (FR)**

(74) Mandataire: **Ipside**
**Tour Trinity**
**1 B Place de la Défense**
**92400 Courbevoie (FR)**

(56) Documents cités:
**EP-A1- 1 273 265        WO-A1-03/073930**
**WO-A1-2008/086624      WO-A2-2009/063463**

* **TERKELSEN A J ET AL: "Acute pain increases heart rate: Differential mechanisms during rest and mental stress", AUTONOMIC NEUROSCIENCE: BASIC AND CLINICAL, ELSEVIER, AMSTERDAM, NL, vol. 121, no. 1-2, 31 août 2005 (2005-08-31), pages 101-109, XP027679934, ISSN: 1566-0702 [extrait le 2005-08-31]**
* **JEANNE M ET AL: "Variations of the analgesia nociception index during general anaesthesia for laparoscopic abdominal surgery", JOURNAL OF CLINICAL MONITORING AND COMPUTING, KLUWER ACADEMIC PUBLISHERS, DO, vol. 26, no. 4, 28 March 2012 (2012-03-28) , pages 289-294, XP035081923, ISSN: 1573-2614, DOI: 10.1007/S10877-012-9354-0**

- **DE JONCKHEERE J ET AL: "Heart rate variability analysis as an index of emotion regulation processes: Interest of the Analgesia Nociception Index (ANI)", ENGINEERING IN MEDICINE AND BIOLOGY SOCIETY (EMBC), 2013 34TH ANNUAL INTERNATIONAL CONFERENCE OF THE IEEE, IEEE, 28 August 2012 (2012-08-28), pages 3432-3435, XP032463678, ISSN: 1557-170X, DOI: 10.1109/EMBC.2012.6346703**

**Description**

La présente invention concerne un dispositif d'évaluation des besoins en médicaments et ou en soins paramédicaux, un procédé d'évaluation pour la mise en oeuvre du dispositif d'évaluation ainsi qu'un dispositif de délivrance associé.

L'invention trouvera avantageusement application dans le domaine de la prise en charge de la douleur.

La prise en charge de la douleur peut se faire en fonction des cas à partir de techniques paramédicales, par des traitements médicamenteux ou encore par une association des deux.

Dans la présente demande, il faut entendre par l'expression « techniques paramédicales » ou « paramédical », l'ensemble des soins pouvant être apportés à un patient à l'exception des traitements médicaux et chirurgicaux, il s'agit notamment, de manière non limitative, des soins de pédiatrie, de kinésithérapie, de néonatalogie, de neurophysiologie, ou encore de manière plus large de soins basés sur une modification de l'environnement de sorte à améliorer le bien-être du patient.

Le dispositif d'évaluation trouvera une application dans l'aide à la décision sur les besoins et la nature des besoins à apporter à un patient pour limiter ou supprimer sa souffrance.

Le dispositif pourra être avantageusement utilisé lors d'une intervention chirurgicale d'un patient sous anesthésie générale ou locale, en évaluant les besoins en composés analgésiques et/ou hypnotiques.

Toutefois cette application n'est pas limitative et le dispositif pourra également être utilisé plus largement pour la prise en charge de la douleur de patients conscients ou inconscients dans les hôpitaux et les structures de soins ou à domicile.

ARRIERE PLAN DE L'INVENTION

Pour réaliser une prise en charge optimale de la douleur d'un patient, il est nécessaire d'en évaluer précisément et en temps réel le niveau afin de pouvoir répondre de manière rapide et adaptée aux besoins du patient notamment par l'injection de médicaments antalgiques ou la réalisation d'actions antalgiques.

En plus de l'obligation morale et légale de cette prise en charge de la douleur, il est également crucial sur le plan médical d'adapter et de personnaliser la prescription.

En effet, par exemple, lorsque le patient est sous anesthésie, une trop forte dose de composés augmente les risques de toxicité ; à l'inverse, une dose insuffisante peut entraîner lors de la phase de réveil des douleurs ou encore une anxiété importante.

Une dose adaptée et transmise en temps réel permet non seulement de soulager le patient lors de l'opération mais encore de réduire les risques de complication postopératoire, et d'améliorer la phase de réveil.

Un premier moyen connu d'évaluation est l'appréciation par le patient lui-même du niveau de douleur ressentie sur une réglette graduée dite Echelle Visuelle Analogique (EVA). Cependant cette évaluation suppose un patient conscient et lucide, et même dans ces circonstances l'évaluation reste ponctuelle et subjective.

On connaît également des dispositifs d'évaluation basés à la fois sur des modèles pharmaco-cinétiques et sur la surveillance de paramètres tels que la fréquence cardiaque et la tension artérielle. Ces dispositifs ne sont toutefois pas satisfaisants en ce sens que les modèles pharmaco-cinétiques ne peuvent s'approcher suffisamment de l'état réel d'un patient, qui dépend de nombreux paramètres propres à chaque individu.

Par ailleurs, dans ces dispositifs, les paramètres physiologiques enregistrés ne sont pas spécifiques des niveaux de douleur mais peuvent traduire d'autres phénomènes.

On connaît également des dispositifs d'évaluation de la douleur basés sur la variabilité de la fréquence cardiaque. Différentes études ont en effet montré la relation entre la variabilité de la fréquence cardiaque et l'activité des systèmes sympathique et parasympathique d'un individu.

En d'autres termes, il a été démontré que l'analyse de la variabilité de la fréquence cardiaque permet de rendre compte du niveau de douleur enduré par un patient.

Cette possibilité est particulièrement intéressante pour l'évaluation d'un patient incapable de communiquer, par exemple un patient inconscient, ou encore un enfant ou encore un malade souffrant de désordres mentaux.

Cette possibilité est également importante puisqu'elle permet de programmer les soins et la nature des soins en fonction du niveau de douleur. Par exemple en néonatalogie cela permet d'éviter ou de différer certains soins douloureux lorsque l'enfant est en situation de confort. Cela permet également d'optimiser la nature des soins en fonction du niveau de douleur et par exemple d'appliquer certains soins médicamenteux (morphinique, paracétamol...) ou non médicamenteux (cocooning, peau à peau, administration de saccharose...) ou encore de modifier l'environnement de l'enfant (bruit, température, lumière...) .

Cette possibilité est également importante pour mesurer avec fiabilité le niveau de douleur, d'autant que la douleur est ressentie de manière très différente d'un patient à l'autre.

Plus précisément, il a été constaté que les fluctuations de la variabilité de la fréquence cardiaque au-delà de 0.15 Hz sont exclusivement dues à l'influence du système parasympathique, ainsi, les phénomènes douloureux, de peur ou encore de stress chez l'adulte se traduisent par une diminution de la variabilité de la fréquence cardiaque haute fréquence c'est-à-dire au-delà de 0.15Hz.

La demanderesse a notamment déposés les demandes suivantes FR 2 821 460, FR 2 840 187, FR 2 864 388 et EP 1 804 655 qui décrivent des dispositifs et procédés permettant notamment de déterminer un indice correspondant au niveau de douleur d'un patient en fonction de la variabilité de la fréquence cardiaque.

Cet indice dit ANI (Analgesia Nociception Index) est fiable et précis, toutefois, il ne permet pas de rendre compte de deux éléments importants et nécessaires pour l'évaluation de la quantité de composés analgésiques et/ou hypnotiques à administrer.

En effet, cet indice ne permet pas directement de différentier le type de douleur ressenti par le patient, à savoir, une douleur aigue ou ponctuelle d'une douleur prolongée. Par ailleurs cet indice ne permet pas non plus de dégager directement une tendance quant à l'évolution de la douleur du patient qui est également nécessaire pour évaluer la quantité de composés à administrer.

Le document EP1273265 décrit un dispositif et procédé permettant la détermination d'un indice correspondant au niveau de douleur d'un patient en fonction de la variabilité de la fréquence cardiaque.

La présente invention se situe dans le domaine de l'évaluation de la douleur basé sur l'analyse de la variabilité de la fréquence cardiaque haute fréquence.

OBJET DE L'INVENTION

La présente invention a pour but de proposer un dispositif d'évaluation des besoins en médicaments ou en soins paramédicaux permettant de déterminer en temps réel le niveau de douleur d'un patient et de prévoir une tendance quant à son évolution à court ou moyen terme.

La présente invention a également pour objet de présenter un dispositif d'évaluation des besoins en composés analgésiques et/ou hypnotiques d'un patient placé sous anesthésie ou sédation permettant une évaluation tenant compte de la tendance sur laquelle se situe le patient.

Un autre objet de la présente invention est de proposer un dispositif d'évaluation des besoins en composés analgésiques et/ou hypnotiques d'un patient placé sous sédation ou anesthésie dans lequel l'évaluation permet de proposer une administration de composés différente en fonction du type de douleur subie par le patient.

Un autre objet de la présente invention est de proposer un dispositif d'évaluation comportant des moyens de communication fiables et en temps réel avec l'anesthésiste.

Un autre objet de la présente invention est de proposer un dispositif de délivrance de composés analgésiques et/ou hypnotiques intégrant un dispositif d'évaluation et permettant une injection automatique ou semi-automatique de composés au patient.

RESUME DE L'INVENTION

L'invention est défini sur les revendications 1 et 9.

A cet effet l'invention concerne un dispositif d'évaluation des besoins en médicaments ou en soins paramédicaux d'un patient avec :

- des moyens de mesure (5) de la variabilité cardiaque entre 0.15Hz et 4Hz,

- des premiers moyens de traitement (6) des données mesurées selon une fenêtre glissante permettant le calcul d'un indice ANI relatif au niveau de douleur du patient caractérisé en ce qu'il comprend en outre :

- des seconds moyens de traitement (7) à partir de l'indice ANI permettant le calcul d'un indice MC, représentatif de la moyenne de l'indice ANI sur une période dite courte, et d'un indice ML, représentatif de la valeur moyenne de l'indice ANI sur une période dite longue, ainsi que les pentes respectivement PC et PL desdits indices MC et ML, les indices et les pentes correspondant au niveau de douleur d'un patient et à son évolution,

- des moyens d'enregistrement (8) de données relatives au patient,

- des troisièmes moyens de traitement (9) permettant de déterminer, en fonction des indices MC et ML de leurs pentes PC et PL et des données relatives au patient, le besoin en composés analgésiques et/ou hypnotiques et ou en soins paramédicaux devant être administrés audit patient.

L'invention vise également de manière plus spécifique un dispositif d'évaluation des besoins en composés analgésiques et/ou hypnotiques d'un patient placé sous sédation ou anesthésie comportant :

- des moyens de mesure de la variabilité cardiaque entre 0.15Hz et 4Hz,

- des premiers moyens de traitement des données mesurées selon une fenêtre glissante permettant le calcul d'un indice (ANI) relatif au niveau de douleur du patient et tel que, selon l'invention le dispositif d'évaluation comprend en outre :

- des seconds moyens de traitement à partir de l'indice ANI permettant le calcul d'un indice MC, représentatif de la moyenne de l'indice ANI sur une période dite courte, et d'un indice ML, représentatif de la valeur moyenne de l'indice ANI sur une période dite longue, ainsi que les pentes respectivement PC et PL desdits indices MC, ML, les indices MC et ML et les pentes correspondant au niveau de douleur d'un patient et à son évolution,

- des moyens d'enregistrement de données relatives au patient,

- des troisièmes moyens de traitement permettant de déterminer, en fonction des indices MC et ML de leurs pentes PC et PL et des données relatives au patient, le besoin en composés analgésiques et/ou hypnotiques devant être administrée audit patient.

L'invention a également pour objet un procédé d'évaluation pour la mise en oeuvre dudit dispositif d'évaluation ainsi qu'un dispositif de délivrance de composés analgésiques et/ou hypnotiques intégrant ledit dispositif d'évaluation.

BREVE DESCRIPTION DES DESSINS

La présente invention sera mieux comprise à la lecture d'un exemple détaillé de réalisation en référence aux dessins annexés, fournis à titre d'exemple non limitatif, parmi lesquels :

- la figure 1 représente un exemple de réalisation schématique d'un dispositif de délivrance conforme à l'invention,

- la figure 2 représente un exemple de réalisation d'interface graphique pour le dispositif d'évaluation,

- la figure 3 représente un exemple de réalisation d'un algorithme d'évaluation conforme à l'invention,

- la figure 4 représente un type particulier de courbe affichée par l'interface graphique.

DESCRIPTION DETAILLEE DE L'INVENTION

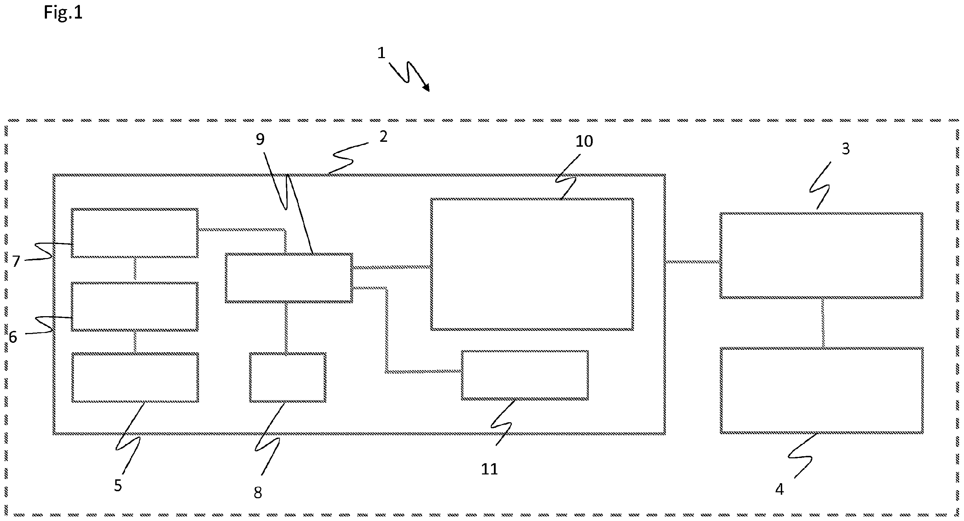

En se reportant principalement à la figure 1 on voit représenté, sous forme schématique, un dispositif de délivrance 1 de composé, ce dispositif de délivrance 1 intègre un dispositif d'évaluation 2, des moyens de commande 3 et un pousse seringue 4.

Il est important de noter à ce niveau que l'invention vise principalement le dispositif d'évaluation 2. Ce dispositif d'évaluation 2 peut en effet être utilisé indépendamment du dispositif de délivrance 1. Notamment, ce dispositif d'évaluation 2 pourra être utilisé en affichant des données relatives à la douleur ressentie par le patient, la tendance sur laquelle il se trouve et une aide pour la prescription de composés.

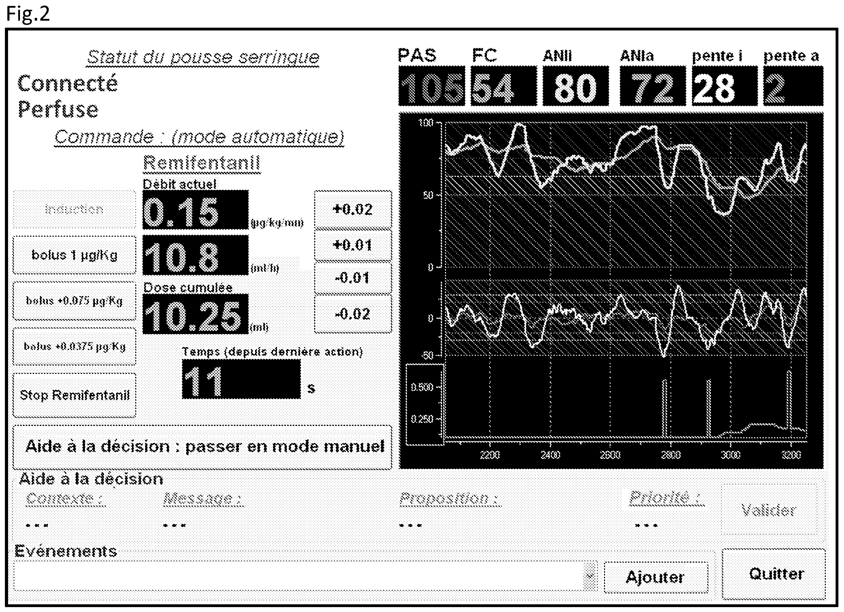

A ce sujet il est intéressant de se reporter à la figure 2 représentant un exemple de réalisation d'une interface graphique permettant l'affichage de ces données.

Les données affichées par l'interface sont alors exploitées par le personnel soignant et plus précisément par l'anesthésiste. Ce dernier utilise l'aide apportée par le dispositif d'évaluation 2 pour adapter la quantité et la nature des composés à injecter au patient.

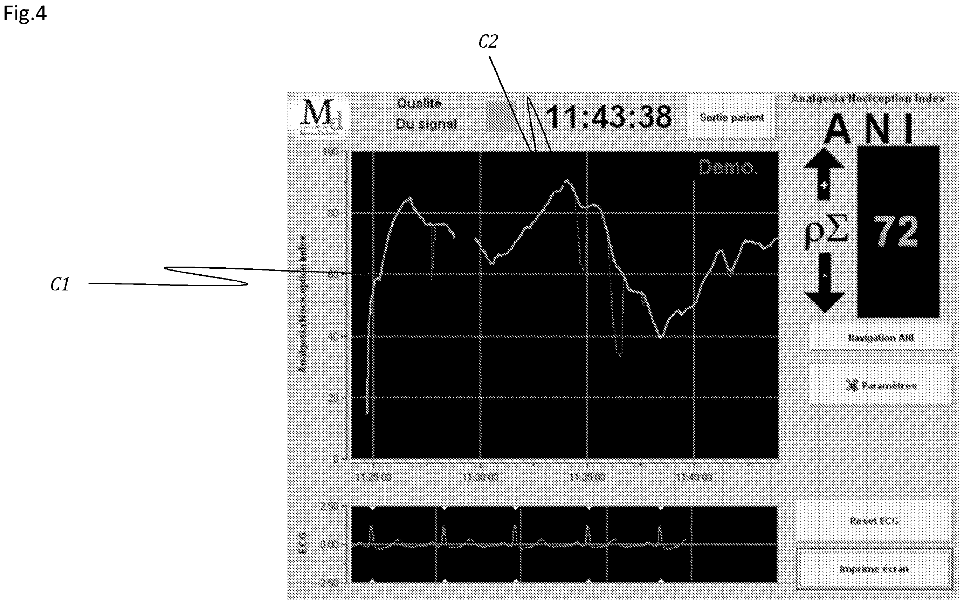

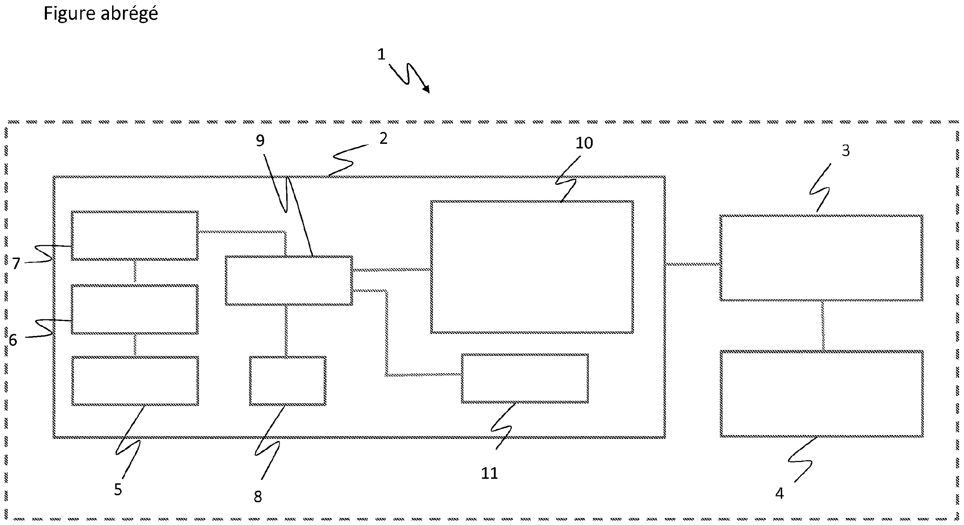

En se reportant à la figure 4, on voit représenté différentes courbes à savoir une première courbe C1 correspondant aux valeurs de l'indice ANI sur une période longue ML et une seconde courbe c2 correspondant aux valeurs de l'indice ANI sur une période courte MC. La seconde courbe c2 n'apparaît sur l'interface graphique qu'en cas de dépassement de seuil correspondant à l'apparition d'une douleur aigue. Ce type d'affichage, ne laissant figurer que les informations pratiques, est particulièrement intéressant puisque le clinicien peut visualiser en direct l'évolution sur une période longue de l'indice de douleur et également agir rapidement en cas d'affichage de la seconde courbe c2 représentative d'une douleur subite aigue.

Le dispositif d'évaluation 2 des besoins en composés analgésiques et/ou hypnotiques d'un patient placé sous anesthésie ou sédation comprend des moyens de mesure 5 de la variabilité cardiaque entre 0.15Hz et 4Hz. Cette mesure peut être acquise notamment à partir d'un électrocardiographe (ECG), toutefois d'autres dispositifs connus de l'homme du métier pourront également être utilisés et, par exemple, à l'aide d'un oxymètre. Le dispositif d'évaluation 2 comprend également des premiers moyens de traitement 6 des données mesurées selon une fenêtre glissante permettant le calcul d'un indice (ANI) relatif au niveau de douleur du patient.

Le niveau de douleur dit ANI est obtenu à partir d'un des procédés d'analyse sur la variabilité du rythme cardiaque connus. Ces procédés consistent à construire à partir de la mesure de la variabilité cardiaque une série dite RR constituée d'une pluralité d'échantillons représentant les intervalles de temps séparant deux battements cardiaques successifs puis à procéder à une analyse de la variabilité de la série RR.

De manière avantageuse le niveau de douleur ANI est calculé dans le dispositif d'évaluation 2 selon le procédé décrit dans la demande WO 2006/032739.

Le dispositif d'évaluation 2 comprend également des seconds moyens de traitement 7 à partir de l'indice ANI permettant le calcul de deux sous indices MC et ML. Le premier indice MC est représentatif de la moyenne de l'indice ANI sur une période dite courte, tandis que le second indice ML est représentatif de la valeur moyenne de l'indice ANI sur une période dite longue.

Les seconds moyens de traitement 7 permettent également le calcul des pentes dites PC et PL desdits indices MC, ML.

Le dispositif d'évaluation 2 comprend également des moyens d'enregistrement 8 de données relatives au patient. Ces moyens d'enregistrement 8 peuvent être réalisés à partir de capteurs déjà présents sur le marché. De manière avantageuse ces moyens d'enregistrement 8 permettent la mesure de la fréquence cardiaque FC et de la pression artérielle systolique PAS. Ces paramètres ne sont pas utilisés directement dans le calcul de la quantité de composés optimale à administrer au patient mais sont utilisés de sorte à constituer des sécurités, le dispositif d'évaluation 2 ne réalise pas d'évaluation lorsque certains seuils correspondant à ces paramètres sont dépassés.

Dans d'autres modes de réalisation, d'autres paramètres physiologiques pourraient être utilisés en remplacement ou additionnellement à la fréquence cardiaque et à la pression artérielle systolique.

Il est également envisageable, bien que ce mode présenterait l'inconvénient d'une sécurité dégradée, de n'utiliser qu'un seul paramètre physiologique.

Il est aussi envisageable d'enregistrer par les moyens d'enregistrement 8 d'autres types de données relatives au patient tels que le poids, la taille, l'âge ou encore le sexe du patient. A cet effet les moyens d'enregistrement 8 comprendront avantageusement un clavier, non représenté dans les figures annexées.

Le dispositif d'évaluation 2 comporte également des troisièmes moyens de traitement 9 permettant de déterminer, en fonction des sous indices MC et ML de leurs pentes PC et PM et des données relatives au patient, la quantité de composés analgésiques et/ou hypnotiques devant être administrée audit patient.

Avantageusement le dispositif d'évaluation 2 comporte en outre des moyens d'affichage 10 permettant de transmettre sur un écran les informations nécessaires à l'anesthésiste.

Le dispositif d'évaluation 2 comporte également des moyens d'alerte 11 permettant de transmettre, notamment sous la forme d'un signal sonore ou lumineux, une urgence relative à l'état du patient.

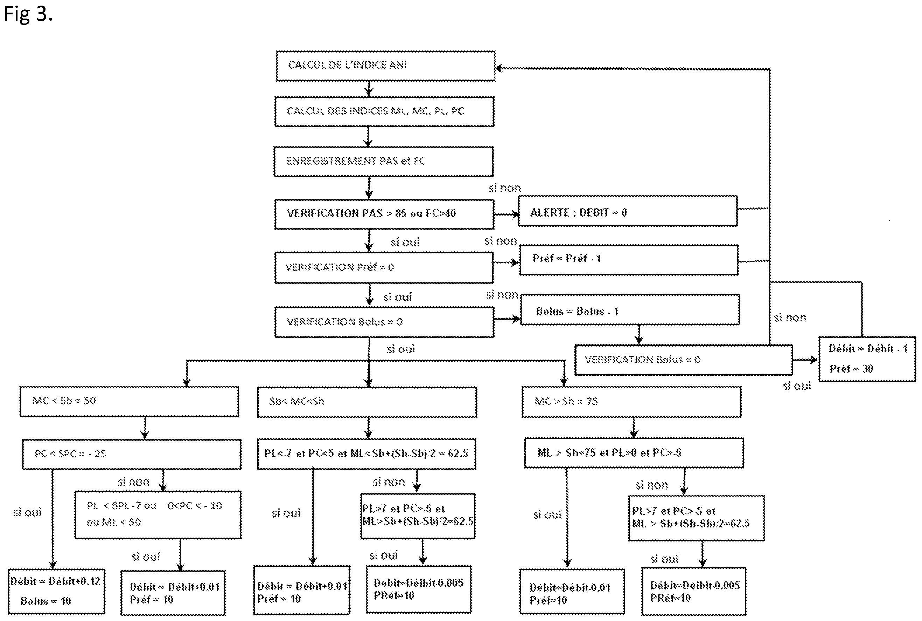

En se reportant cette fois à la figure 3, on voit représenté un exemple préféré de réalisation des second et troisième moyens de traitement permettant d'aboutir à une évaluation des composés à prescrire ou injecter au patient. Cet exemple est présenté sous la forme d'un algorithme.

Cet algorithme utilise notamment l'indice ANI, les indices MC et ML et les pentes PC et PL de ces indices.

Les indices MC et ML sont calculés à partir des différentes valeurs de l'ANI pendant une durée donnée et permettent de limiter l'impact de la variabilité instantanée de la mesure de l'ANI due à la méthode de calcul et également de dégager des tendances dans l'évolution de la douleur du patient.

Plus précisément, le calcul des indices MC et ML est réalisé à partir d'une moyenne des valeurs des indices ANI sur deux périodes différentes à savoir une période courte et une période longue.

De manière avantageuse, la période courte correspond à une fenêtre glissante de 45 à 90 secondes et de préférence de l'ordre de 60 secondes, tandis que la période longue correspond à une fenêtre glissante de 90 à 240 secondes et de préférence de l'ordre de 180 secondes.

De manière avantageuse, la période prise en compte pour le calcul de ML est sensiblement de l'ordre de 1.5 à 3 fois la période utilisée pour le calcul de MC.

La pente PC est calculée en degrés à partir de deux valeurs MC séparés d'une durée donnée selon la formule suivante :

$$PC = (180/\pi) \, arctg((MC\,(t2) - MC(t1))/\,(t2-t1))$$

Avantageusement l'intervalle de temps entre t1 et t2 est de l'ordre de 15 à 45 secondes et de préférence égal à 30 secondes.

La pente PL est calculée en degrés de manière analogue à celle de PC selon la formule suivante :

$$PL= (180/\pi)arctg((ML(t2) - ML(t1))/(t2-t1))$$

Là encore l'intervalle de temps séparant t1 et t2 est de l'ordre de 15 à 45 secondes et de préférence égal à 30 secondes.

Les seconds moyens de traitement comportent différents seuils, permettant d'évaluer les besoins en composés du patient.

Une première catégorie de seuils concerne l'indice MC, il s'agit d'un seuil bas Sb et d'un seuil haut Sh.

Dans l'exemple préféré de réalisation des figures annexées, le niveau de douleur est compris entre 0, correspondant à un niveau de douleur extrême ou maximum, et 100, correspondant à une absence de douleur.

Dans cet exemple, on donne avantageusement la valeur de 50 pour le seuil bas Sb et 75 pour le seuil haut Sh.

Une seconde catégorie de seuils concerne les indices PC et PL correspondant aux pentes des courbes MC et ML, il s'agit de deux seuils SPC et SPL, de préférence SPC est fixé à 25 et le SPL est fixée à 7.

Une troisième catégorie de seuil correspond à une durée minimale, dite période réfractaire, après une administration ou un changement de débit du composé afin d'en évaluer les effets, ce seuil Préf est réglable en fonction du composé utilisé.

Une quatrième catégorie de seuil correspond à des paramètres physiologiques du patient, dans l'exemple de réalisation dans lequel les paramètres pris en compte sont la fréquence cardiaque FC et la pression artérielle et avantageusement la pression artérielle systolique ou PAS. Dans ce mode de réalisation préférée, on fixe un seuil inférieur SFC pour la fréquence cardiaque avantageusement de 40 battements par minute correspondant à une bradycardie.

On fixe également un seuil inférieur SPAS pour la pression artérielle systolique avantageusement à 80 mm Hg correspondant à un passage du patient en hypotension.

Comme représenté à la figure 3, le procédé d'évaluation pour la mise en oeuvre du dispositif comprend une première étape de calcul de l'indice ANI par les premiers moyens de traitement 6.

Le procédé comprend ensuite une seconde étape de calcul des indices ML et MC et des pentes associées PL et PC avantageusement selon les formules détaillées plus haut. Cette seconde étape est réalisée par les seconds moyens de traitement 7.

Le procédé d'évaluation comprend ensuite une étape de mesure des paramètres physiologiques par les moyens d'enregistrement 8.

A partir des résultats obtenus dans les deux premières étapes de traitement et de l'étape de mesure, le procédé réalise une troisième étape de traitement décrite ci-après.

La troisième étape de traitement est réalisée par les troisièmes moyens de traitement 9. Dans cette étape, on réalise une comparaison entre les mesures des paramètres FC et PAS et leurs valeurs seuils respectivement SFC et SPAS. Lorsque les mesures sont inférieures aux valeurs seuils SFC et SPAS, le calcul de l'évaluation est stoppé.

De manière avantageuse une alerte est émise par les moyens d'alerte 11, le débit de composé injecté est passé à zéro et une temporisation est enclenchée de sorte à relancer le procédé à la fin de la temporisation. Lorsque les valeurs de FC et PAS sont supérieures aux valeurs seuils SFC et SPAS, la troisième étape de traitement continue.

Les troisièmes moyens de traitement 9 vérifie si la période réfractaire Préf est à zéro seconde dans le cas contraire le calcul d'évaluation est relancé.

Lorsque la période Préf est à zéro seconde, les troisièmes moyens de traitement 9 vérifient le solde de bolus. En effet lorsqu'une dose de médicament est administrée rapidement au patient, le niveau de bolus passe à 10, correspondant à la durée de l'administration et lorsque le niveau repasse à 0, après l'administration, les troisièmes moyens de traitement 9 démarrent une nouvelle période réfractaire Préf.

Lorsque le niveau de bolus est à zéro, les troisièmes moyens de traitement 9 comparent le niveau de ML aux niveaux de seuil bas et haut respectivement Sb et Sh.

Cette comparaison permet de placer le patient dans une des trois catégories dites analgésie insuffisante, adaptée ou importante.

Le traitement s'effectue ensuite en fonction du niveau de MC par rapport à ces seuils Sb et Sh :

- MC inférieur à 50 (Sb)

Lorsque l'indice MC représentatif de la douleur ressentie par le patient sur une moyenne courte est inférieur à 50 on considère que l'analgésie est insuffisante.

La dose à évaluer peut être administrée soit de manière étalée dans le temps soit de manière rapide (ou bolus).

Le type d'administration dépend du type de douleur ressentie par le patient et plus précisément l'administration sera étalée dans le temps en cas de douleur prolongée et un bolus en cas de douleur aigué.

Selon l'invention, la douleur est considérée comme aigue lorsque la valeur de l'indice représentatif de la pente courte est inférieure à la valeur seuil SPC. De manière avantageuse cette valeur de seuil est fixée à -25. Dans ce cas de figure, et en se reportant une nouvelle fois à la figure 3, la consigne de débit est augmentée fortement durant 10 secondes suivie d'une période réfractaire.

A titre d'exemple, lorsque le composé utilisé est du remifentanil de concentration à 0.25pg/ml, le débit est augmenté en fonction du poids du patient à 0.12 pg/kg/mn pendant 10 secondes.

Lorsque la valeur de PC mesurée est supérieure à la valeur seuil SPC, on compare ensuite les valeurs PL, PC et ML. La valeur PL est comparée au seuil SPL si cette valeur PL est inférieure au seuil, on considère qu'il y a défaut d'analgésie prolongée. De même lorsque la valeur de PC est légèrement négative et avantageusement comprise entre 0 et -7. On considère également qu'il y a défaut d'analgésie prolongée lorsque la valeur de ML est inférieure à 50.

Dans le cas d'un défaut d'analgésie prolongée la consigne de débit est augmentée faiblement, cette action étant suivie d'une période réfractaire Préf.

A titre d'exemple, lorsque le composé utilisé est du remifentanil de concentration à 25pg/ml, le débit est augmenté en fonction du poids du patient à 0.01 pg/kg/mn.

Si l'état du patient n'est pas considéré comme en état de douleur aigue ou encore de défaut d'analgésie prolongée, le dispositif d'évaluation ne modifie pas la consigne de débit et le procédé d'évaluation est relancé.

- ML compris entre 50 (Sb) et 75 (Sh)

Lorsque l'indice MC représentatif de la douleur ressentie par le patient sur une moyenne courte est compris entre 50 et 75, on considère que l'analgésie est adaptée. Toutefois, en fonction des sous indices représentatifs de l'évolution du patient, le dispositif d'évaluation pourra donner des consignes d'augmentation ou de réduction du débit suivies d'une période réfractaire pour en évaluer les effets.

Dans un premier temps, les troisièmes moyens de traitement 9 comparent les valeurs des indices ML, PL et PC. Lorsque la valeur de ML est inférieure à une valeur intermédiaire avantageusement 62.5, calculé avec la formule Sb + (Sh-Sb)/2 et que la valeur de PL est inférieure à la valeur seuil SPL et enfin que la valeur de PC est inférieure à une valeur intermédiaire avantageusement 5, le dispositif d'évaluation 2 considère que le patient a une évolution nécessitant une augmentation de la consigne de débit, l'envoi de cette consigne est suivi d'une période réfractaire Préf.

A titre d'exemple, lorsque le composé utilisé est du remifentanil de concentration à 25pg/ml, le débit est augmenté en fonction du poids du patient à 0.01 pg/kg/mn.

Dans le cas contraire le dispositif d'évaluation 2 compare à nouveau les valeurs de ML, PL et PC. Lorsque la valeur de ML est supérieure à une valeur intermédiaire avantageusement 62.5 et que la valeur de PL est supérieure à une autre valeur intermédiaire avantageusement 7 et que la valeur de PC est supérieure à une valeur intermédiaire avantageusement de -5, le dispositif d'évaluation 2 considère que la consigne de débit doit être réduite. L'envoi de cette consigne est suivi d'une période réfractaire Préf.

A titre d'exemple, lorsque le composé utilisé est du remifentanil de concentration à 25pg/ml, le débit est diminué en fonction du poids du patient à -0.005 pg/kg/mn.

Lorsque les valeurs ne sont pas dans les deux cas de figure précités, le dispositif d'évaluation ne donne aucune consigne de débit et le procédé d'évaluation est relancé.

- ML supérieur à 75 (Sh)

Lorsque l'indice MC représentatif de la douleur ressentie par le patient sur une moyenne courte est supérieur au seuil haut Sh, avantageusement de 75, on considère que l'analgésie est importante et le dispositif d'évaluation 2 analyse si ce niveau est trop élevé et nécessite une réduction du débit.

On prévoit de manière avantageuse deux niveaux de réduction du débit.

Dans un premier temps, les troisièmes moyens de traitement 9 comparent les valeurs des indices ML, PL et PC. Lorsque la valeur de ML est inférieure à une valeur intermédiaire avantageusement 75 et que la valeur de PL est supérieure à 0 et enfin que la valeur de PC est inférieure à une valeur intermédiaire avantageusement -5, le dispositif d'évaluation 2 considère que le patient a une évolution nécessitant une réduction relativement importante de la consigne de débit, l'envoi de cette consigne est suivi d'une période réfractaire Préf.

A titre d'exemple, lorsque le composé utilisé est du remifentanil de concentration à 25pg/ml, le débit est diminué en fonction du poids du patient à -0.01 pg/kg/mn.

Dans le cas contraire le dispositif d'évaluation 2 compare à nouveau les valeurs de ML, PL et PC. Lorsque la valeur de ML est supérieure à une valeur intermédiaire avantageusement 65 et que la valeur de PL est supérieure à une autre valeur intermédiaire avantageusement 7 et que la valeur de PC est supérieure à une valeur intermédiaire avantageusement de -5, le dispositif d'évaluation 2 considère que la consigne de débit doit être réduite mais moins rapidement que dans le cas précédent. L'envoi de cette consigne est suivi d'une période réfractaire Préf.

Là encore, lorsque les valeurs ne sont pas dans les deux cas de figure précités, le dispositif d'évaluation ne donne aucune consigne de débit et le procédé d'évaluation est relancé.

Le dispositif d'évaluation 2, décrit ci-dessus, prévoit un certain nombre de seuils, toutefois l'ensemble de ces seuils peut être modifié ou paramétré en fonction du patient et/ou du type de produit utilisé.

Par ailleurs, différentes étapes réalisées par les troisièmes moyens de traitement 9 peuvent être exécutées dans un ordre différent, par exemple l'étape de comparaison des mesures physiologiques pourrait être déplacée en fin d'évaluation.

Il est également important de noter que les premier, second, et troisième moyens de traitement peuvent être réalisés par une seule unité centrale.

Ce dispositif d'évaluation 2 permet par conséquent de constituer une aide directe, fiable et précise pour l'anesthésiste. Le dispositif de délivrance 1 intégrant le dispositif d'évaluation 2 constitue une solution optimale pour la délivrance automatique ou semi-automatique de composés au patient notamment durant les opérations de chirurgie sous anesthésie.

Bien entendu, d'autres caractéristiques de l'invention auraient également pu être envisagées sans pour autant sortir du cadre de l'invention définie par les revendications ci-après.

## Revendications

1. Dispositif d'évaluation des besoins en médicaments ou en soins paramédicaux d'un patient **caractérisé en ce qu'**il comprend :

   - des moyens de mesure (5) de la variabilité cardiaque entre 0.15Hz et 4Hz,
   - des premiers moyens de traitement (6) des données mesurées selon une fenêtre glissante permettant le calcul d'un indice ANI relatif au niveau de douleur du patient, obtenu à partir d'un procédé d'analyse de la variabilité cardiaque du patient, consistant à construire à partir de la mesure de la variabilité cardiaque une série dite RR, puis à procéder à une analyse de la variabilité de la série RR, **caractérisé en ce qu'**il comprend en outre :
   - des seconds moyens de traitement (7) à partir de l'indice ANI permettant le calcul d'un indice MC, représentatif de la moyenne de l'indice ANI sur une période dite courte, et d'un indice ML, représentatif de la valeur moyenne de l'indice ANI sur une période dite longue, ainsi que les pentes respectivement PC et PL desdits indices MC et ML, les i'dices et les pentes correspondant au niveau de douleur d'un patient et à son évolution,
   - des moyens d'enregistrement (8) de données relatives au patient,
   - des troisièmes moyens de traitement (9) permettant de déterminer, en fonction des indices MC et ML, de leurs pentes PC et PL et des données relatives au patient, le besoin en composés analgésiques et/ou hypnotiques et/ou en soins paramédicaux devant être administrés audit patient.

2. Dispositif d'évaluation selon la revendication précédente permettant l'évaluation des besoins en composés analgésiques et/ou hypnotiques d'un patient placé sous sédation ou anesthésie ou les troisièmes moyens de traitement (9) permettent de déterminer, en fonction des indices MC et ML de leurs pentes PC et PL et des données relatives au patient, le besoin en composés analgésiques et/ou hypnotiques devant être administrée audit patient.

3. Dispositif d'évaluation selon l'une ou l'autre des revendications 1 et 2 dans lequel le calcul des indices MC et ML est réalisé à partir d'une moyenne des valeurs des indices ANI sur deux périodes glissantes différentes à savoir une période courte et une période longue.

4. Dispositif d'évaluation selon la revendication 3 dans lequel la période longue est de 1.5 à 3 fois la période courte.

5. Dispositif d'évaluation selon l'une quelconque des revendications précédentes dans lequel les valeurs de PC et PL sont calculés selon les formules suivantes :

$$PC = (180/n) \; arctg((MC(t2) - MC(tl))/(t2-tl))$$

et

$$PL = (180/n) \; arctg((ML(t2) - ML(t1))/ \; (t2-tl)).$$

**6.** Dispositif d'évaluation selon l'une quelconque des revendications précédentes dans lequel les moyens d'enregistrement (8) permettent l'enregistrement de la fréquence cardiaque et/ou la pression artérielle des patients, et tels que les troisièmes moyens de traitement (9) stoppent l'évaluation en cas de dépassement des seuils SFC et SPAS relatifs à ces paramètres.

**7.** Dispositif d'évaluation selon l'une quelconque des revendications précédentes comportant des moyens d'affichage (10) permettant d'afficher sur une interface les informations relatives au patient.

**8.** Dispositif d'évaluation selon l'une quelconque des revendications précédentes comportant des moyens d'alerte (11) permettant de transmettre un message d'urgence relatif à l'état du patient.

**9.** Procédé d'évaluation pour la mise en œuvre du dispositif selon l'une quelconque des revendications précédentes dans lequel on réalise les étapes suivantes :

   - une étape de mesure de la variabilité cardiaque entre 0.15Hz et 4Hz,
   - une première étape de traitement des données mesurées selon une fenêtre glissante permettant le calcul d'un indice ANI relatif au niveau de douleur du patient, obtenu à partir d'un procédé d'analyse de la variabilité cardiaque du patient, consistant à construire à partir de la mesure de la variabilité cardiaque une série dite RR, puis à procéder à une analyse de la variabilité de la série RR, **caractérisé en ce qu'**il comprend en outre :
   - une seconde étape de traitement à partir de l'indice ANI permettant le calcul d'un indice MC, représentatif de la moyenne de l'indice ANI sur une période dite courte, et d'un indice ML, représentatif de la valeur moyenne de l'indice ANI sur une période dite longue, ainsi que les pentes respectivement PC et PL desdits indices MC, ML, les indices MC e ML et les pentes correspondant au niveau de douleur d'un patient et à son évolution,
   - une étape d'enregistrement de données relatives au patient,
   - une troisième étape de traitement permettant de déterminer, en fonction des indices MC et ML de leurs pentes PC et PL et des données relatives au patient, le besoin en composés analgésiques et/ou hypnotiques devant être administrée audit patient.

**10.** Procédé d'évaluation selon la revendication 9 dans lequel la troisième étape comprend une étape de placement du patient dans une des trois catégories dites analgésie insuffisante, adaptée ou importante, en fonction de la valeur de l'indice MC par rapport à des seuils Sb et Sh.

**11.** Procédé d'évaluation selon la revendication 10 dans lequel la troisième étape comprend, après l'étape de placement dans une des trois catégories, une étape d'évaluation de la consigne en fonction d'au moins un des indices ML, PL et PC.

**12.** Procédé d'évaluation selon l'une quelconque des revendications 9 à 11 précédentes dans lequel la consigne d'administration d'un composé se fait sous la forme d'un bolus lorsque la valeur de l'indice PC est inférieure à une valeur seuil SPC.

**13.** Procédé d'évaluation selon l'une quelconque des revendications 9 à 12 dans lequel tout ou partie des seuils est ajustable.

**14.** Dispositif de délivrance d'un composé hypnotique et/ou analgésique comprenant un dispositif d'évaluation selon l'une quelconque des revendications 2 à 8 précédentes, associé à des moyens de commande (3) et à un pousse-seringue (4).

**Patentansprüche**

**1.** Vorrichtung zur Beurteilung des Bedarfs an Medikamenten oder paramedizinischer Versorgung eines Patienten, **dadurch gekennzeichnet, dass** sie Folgendes umfasst:

   - ein Messmittel (5) für die Herzratenvariabilität zwischen 0,15 Hz und 4 Hz,
   - ein erstes Verarbeitungsmittel (6) für über ein gleitendes Zeitfenster gemessene Daten, die das Berechnen eines ANI-Index in Bezug auf das Schmerzniveau des Patienten ermöglichen, das aus einem Analyseverfahren der Herzratenvariabilität des Patienten erhalten wird, das aus dem Messen der Herzratenvariabilität in einer sogenannten RR-Reihe und anschließend dem Durchführen einer Analyse der Variabilität der RR-Reihe besteht,

**dadurch gekennzeichnet, dass** es ferner Folgendes umfasst:

- ein zweites Verarbeitungsmittel (7), dass das Berechnen, aus dem ANI-Index, eines MC-Index, der repräsentativ für den durchschnittlichen ANI-Index über einen kurzen Zeitraum ist, und eines ML-Index, der repräsentativ für den Durchschnittswert des ANI-Index über einen langen Zeitraum ist, sowie der PC- bzw. PL-Steigungen der MC- und ML-Indizes, wobei die Indizes und die Steigungen dem Schmerzniveau eines Patienten und seiner Entwicklung entsprechen, ermöglicht,
- ein Aufzeichnungsmittel (8) für Daten in Bezug auf den Patienten,
- ein drittes Verarbeitungsmittel (9), das es ermöglicht, in Abhängigkeit von MC- und ML-Indizes, ihrer PC- und PL-Steigungen und Daten in Bezug auf den Patienten den Bedarf an analgetischen und/oder hypnotischen Verbindungen oder paramedizinischer Versorgung, die dem Patienten verabreicht werden muss, zu bestimmen.

2. Beurteilungsvorrichtung nach dem vorhergehenden Anspruch, die die Beurteilung des Bedarfs an analgetischen und/oder hypnotischen Verbindungen für einen Patienten, der unter Sedierung oder Anästhesie steht, ermöglicht, wobei das dritte Verarbeitungsmittel (9) es ermöglicht, in Abhängigkeit von den MC- und ML-Indizes, ihrer PC- und PL-Steigungen und den Daten in Bezug auf den Patienten den Bedarf an analgetischen und/oder hypnotischen Verbindungen, die dem Patienten verabreicht werden müssen, zu bestimmen.

3. Beurteilungsvorrichtung nach einem der Ansprüche 1 und 2, wobei das Berechnen der MC- und ML-Indizes anhand eines Durchschnitts der Werte der ANI-Indizes für zwei verschiedene gleitende Zeiträume, nämlich einen kurzen Zeitraum und einen langen Zeitraum, durchgeführt wird.

4. Beurteilungsvorrichtung nach Anspruch 3, wobei der lange Zeitraum das 1,5- bis 3-Fache des kurzen Zeitraums beträgt.

5. Beurteilungsvorrichtung nach einem der vorhergehenden Ansprüche, wobei die PC- und PL-Werte unter Verwendung der folgenden Formeln berechnet werden:

$$PC = (180/n)\ \text{arctg}((MC(t2) - MC(t1))/(t2-t1))$$

und PL = (180/n) arctg((ML(t2) - ML(t1))/(t2-tl)).

6. Beurteilungsvorrichtung nach einem der vorhergehenden Ansprüche, wobei das Aufzeichnungsmittel (8) das Aufzeichnen der Herzfrequenz und/oder des Blutdrucks von Patienten ermöglicht und das dritte Verarbeitungsmittel (9) das Beurteilen bei Überschreitung der SFC- und SPAS-Schwellenwerte in Bezug auf diese Parameter beendet.

7. Beurteilungsvorrichtung nach einem der vorhergehenden Ansprüche, umfassend ein Anzeigemittel (10), das es ermöglicht, die Informationen in Bezug auf den Patienten auf einer Schnittstelle anzuzeigen.

8. Beurteilungsvorrichtung nach einem der vorhergehenden Ansprüche, umfassend ein Alarmmittel (11), das es ermöglicht, eine Notfallmeldung in Bezug auf den Zustand des Patienten zu versenden.

9. Beurteilungsverfahren zur Ausführung der Vorrichtung nach einem der vorhergehenden Ansprüche, wobei die folgenden Schritte durchgeführt werden:

- ein Schritt des Messens der Herzratenvariabilität zwischen 0,15 Hz und 4 Hz,
- ein erster Schritt des Verarbeitens der über ein gleitendes Zeitfenster gemessenen Daten, die das Berechnen eines ANI-Index in Bezug auf das Schmerzniveau des Patienten ermöglichen, das aus einem Analyseverfahren der Herzratenvariabilität des Patienten erhalten wird, das aus dem Messen der Herzratenvariabilität in einer sogenannten RR-Reihe und anschließend dem Durchführen einer Analyse der Variabilität der RR-Reihe besteht,

**dadurch gekennzeichnet, dass** er ferner Folgendes umfasst:

- einen zweiten Schritt des Verarbeitens aus dem ANI-Index, das das Berechnen eines MC-Index, der repräsentativ für den durchschnittlichen ANI-Index über einen kurzen Zeitraum ist, und eines ML-Index, der repräsentativ für den Durchschnittswert des ANI-Index über einen langen Zeitraum ist, sowie der PC- bzw. PL-Steigungen der MC- und ML-Indizes, wobei die Indizes und die Steigungen dem Schmerzniveau eines Patienten und seiner Entwicklung entsprechen,
- einen Schritt des Aufzeichnens von Daten in Bezug auf den Patienten,
- einen dritten Schritt des Verarbeitens, der es ermöglicht, in Abhängigkeit von den MC- und ML-Indizes, ihrer PC- und PL-Steigungen und den Daten in Bezug auf den Patienten den Bedarf an analgetischen und/oder hypnotischen Verbindungen, die dem Patienten verabreicht werden müssen, zu bestimmen.

10. Beurteilungsverfahren nach Anspruch 9, wobei der dritte Schritt einen Schritt des Einstufens des Patienten in eine von drei Kategorien genannt unzureichende, angepasste oder hohe Analgesie, umfasst, abhängig vom Wert des MC-Index im Vergleich zu Sb- und Sh-Schwellenwerten.

11. Beurteilungsverfahren nach Anspruch 10, wobei der dritte Schritt nach dem Schritt des Einstufens in eine von drei Kategorien einen Schritt des Beurteilens der Vorgabe in Abhängigkeit von mindestens eines der ML-, PL- und PC-Indizes umfasst.

12. Beurteilungsverfahren nach einem der vorhergehenden Ansprüche 9 bis 11, wobei die Vorgabe der Verabreichung einer Verbindung in Form eines Bolus erfolgt, wenn der Wert des PC-Index kleiner als ein SPC-Schwellenwert ist.

13. Beurteilungsverfahren nach einem der Ansprüche 9 bis 12, wobei alle oder ein Teil der Schwellenwerte einstellbar sind.

14. Vorrichtung zur Abgabe einer hypnotischen und/oder analgetischen Verbindung, umfassend eine Beurteilungsvorrichtung nach einem der vorhergehenden Ansprüche 2 bis 8, die in Verbindung mit einem Steuermittel (3) und einer Spritzenpumpe (4) steht.

## Claims

1. A device for assessing the needs for medication or paramedical care of a patient, **characterised in that** it comprises:

   - means (5) for measuring the heart rate variability between 0.15Hz and 4Hz,
   - first means (6) for processing the measured data according to a sliding window enabling the calculation of an index ANI relating to the level of pain suffered by the patient, obtained from a method for analysing the heart rate variability of the patient, consisting in building from the measurement of the heart rate variability a so-called RR series, then proceeding with an analysis of the variability of the series RR,

   **characterised in that** it further comprises:

   - second processing means (7) based on the index ANI enabling the calculation of an index MC, representative of the average of the index ANI over a so-called short period, and of an index ML, representative of the average value of the index ANI over a so-called long period, as well as the slopes PC and PL respectively of said indices MC and ML, the indices and the slopes corresponding to the level of pain suffered by a patient and its evolution,
   - means (8) for recording data relating to the patient,
   - third processing means (9) allowing determining, according to the indices MC and ML, to their slopes PC and PL and to the data relating to the patient, the need for analgesic and/or hypnotic compounds and or paramedical care to be administered to said patient.

2. The assessment device according to the preceding claim, enabling the assessment of the needs for analgesic and/or hypnotic compounds of a patient placed under sedation or anaesthesia, where the third processing means (9) allow determining, according to the indices MC and ML, their slopes PC and PL and the data relating to the patient, the need for analgesic and/or hypnotic compounds to be administered to said patient.

3. The assessment device according to either one of claims 1 and 2, wherein the calculation of the indices MC and ML is carried out based on an average of the values of the indices ANI over two different sliding periods, namely a short period and a long period.

4. The assessment device according to claim 3, wherein the long period is 1.5 to 3 times as long as the short period.

5. The assessment device according to any one of the preceding claims, wherein the values of PC and PL are calculated according to the following formulas:

$$PC = (180/n)arctg((MC(t2) - MC(t1))/(t2 - t1))$$

and

$$PL = (180/n)arctg((ML(t2) - ML(t1))/(t2 - t1))$$

6. The assessment device according to any one of the preceding claims, wherein the recording means (8) enable recording of the heart rate and/or blood pressure of the patients, and such that the third processing means (9) stop the assessment if the SFC and SPAS thresholds related to these parameters are exceeded.

7. The assessment device according to any one of the preceding claims, including display means (10) allowing displaying information relating to the patient on an interface.

8. The assessment device according to any one of the preceding claims, including alert means (11) allowing transmitting an emergency message relating to the condition of the patient.

9. An assessment method for the implementation of the device according to any one of the preceding claims, wherein the following steps are carried out:

   - a step of measuring heart rate variability between 0.15Hz and 4Hz,
   - a first step of processing the measured data according to a sliding window enabling the calculation of an index ANI relating to the level of pain suffered by the patient, obtained from a method for analysing the heart rate variability of the patient, consisting in building from the measurement of the heart rate variability a so-called RR series, then proceeding with an analysis of the variability of the series RR,

   **characterised in that** it further comprises:

   - a second processing step based on the index ANI enabling the calculation of an index MC, representative of the average of the index ANI over a so-called short period, and of an index ML, representative of the average value of the index ANI over a so-called long period, as well as the slopes PC and PL respectively of said indices MC, ML, the indices MC and ML and the slopes corresponding to the level of pain suffered by a patient and its evolution,
   - a step of recording data relating to the patient,
   - a third processing step allowing determining, according to the indices MC and ML, their slopes PC and PL and the data relating to the patient, the need for analgesic and/or hypnotic compounds to be administered to said patient.

10. The assessment method according to claim 9, wherein the third step comprises a step of placing the patient in one of the three categories called insufficient, moderate or significant analgesia, according to the value of the index MC with respect to thresholds Sb and Sh.

11. The assessment method according to claim 10, wherein the third step comprises, after the step of placement in one of the three categories, a step of assessing the instruction according to at least one of the indices ML, PL and PC.

12. The assessment method according to any one of the preceding claims 9 to 11, wherein the instruction for administering a compound is in the form of a bolus when the value of the index PC is lower than a threshold value SPC.

13. The assessment method according to any one of claims 9 to 12, wherein all or part of the thresholds are adjustable.

14. A device for delivering a hypnotic and/or analgesic compound comprising an assessment device according to any one of the preceding claims 2 to 8, associated with control means (3) and with a syringe pump (4).

Fig.1

Fig.2

EP 3 007 614 B1

Fig 3.

Fig.4

Figure abrégé

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2821460
- FR 2840187
- FR 2864388
- EP 1804655 A
- EP 1273265 A
- WO 2006032739 A